# EUROPEAN PATENT SPECIFICATION

(11) **EP 3 845 136 B1**
(45) Date of publication and mention of the grant of the patent: **20.07.2022**
(21) Application number: 18932862.8
(22) Date of filing: 13.11.2018
(51) Int. Cl.: A61B 1/00, A61B 17/34, A61B 17/00, A61B 17/17

(54) **MULTI-CHANNEL WORKING CANNULA**
MEHRKANALIGE ARBEITSKANÜLE
CANULE DE TRAVAIL À CANAUX MULTIPLES

(30) Priority: 05.09.2018 CN 201821448514 U
(43) Date of publication of application: 07.07.2021
(73) Proprietor: Dragon Crown Medical Co., Ltd., Jinan, Shandong 250101 (CN)
(72) Inventor: YANG, Wenzhou, Jinan, Shandong 250101 (CN); PAN, Huihui, Jinan, Shandong 250101 (CN)
(74) Representative: Sun, Yiming
(86) International application number: PCT/CN2018/000387
(87) International publication number: WO 2020/047687

(56) References cited:
- WO-A1-2016/166827
- WO-A1-2017/036989
- WO-A1-2017/109900
- CN-A- 102 613 996
- CN-A- 106 236 210
- CN-A- 107 995 856
- CN-U- 207 024 116
- US-A1- 2009 227 843
- US-A1- 2010 228 092
- US-A1- 2014 046 383
- US-A1- 2015 250 498

## Description

### TECHNICAL FIELD

This application relates to the technical field of medical devices, and in particular to a multi-channel working cannula.

### BACKGROUND

Minimally invasive surgery refers to a surgery performed by use of modern medical instruments such as laparoscope or thoracoscope, as well as a minimally invasive channel device and related equipment. Since the minimally invasive surgery has the advantages of less trauma, light pain, and fast recovery, the concept of "minimally invasive" has penetrated into various fields of surgical operations.

In the prior art, the minimally invasive channel device includes a guide tube and an expansion tube, and the guide tube is arranged inside the expansion tube. In an actual surgery, when it is needed to grind the bones around the affected area, the abrasion drill needs to be inserted along the guide tube to the affected area for bone grinding. because there is only one guide tube in the expansion tube, only the abrasion drill can be inserted at this time, and an endoscope cannot be inserted at the same time, therefore, the abrasion drill is in a blind grinding state during the actual grinding process, so that the abrasion drill cannot always be accurately aligned with the part that needs to be ground during the bone grinding process; at the same time, after the end of the bone grinding, when the endoscope needs to be inserted for observation, it is needed to first remove the abrasion drill from the guide tube, and then insert the endoscope from the guide tube for observation. The operation procedure is cumbersome, which prolongs the operation time and causes a certain injury to the patient.

US 2010/228092 A1 refers to various methods and devices being provided for allowing multiple surgical instruments to be inserted into sealing elements of a single surgical access device. The sealing elements can be movable along predefined pathways within the device to allow surgical instruments inserted through the sealing elements to be moved laterally, rotationally, angularly, and vertically relative to a central longitudinal axis of the device for ease of manipulation within a patient's body while maintaining insufflation.

US 2009/227843 A1 refers to a multi-instrument access device including a base positionable within an opening (e.g. an incision or puncture) formed in a body wall and a dome-shaped seal on the base and positioned such that it is disposed outside the body wall during use. A plurality of instrument ports extend proximally from the seal for receiving instruments to be inserted into the body for use in a procedure. Tubular instrument tubes having pre-curved distal ends may be insertable through the ports for receiving the instruments and for orienting the operative ends of the instruments toward a target site.

US 2015/250498 A1 refers to a trocar including a distal-end tube portion that is disposed inside the body and has a first inner hole through which a medical device can be inserted; a proximal-end tube portion that is disposed outside the body and is connected to the distal-end tube portion, the proximal-end tube portion having a second inner hole that communicates with the first inner hole and has an axis which can be disposed in a direction not parallel to an axis of the first inner hole; and a movement facilitating portion that is disposed on at least one of the inner surface of the first inner hole and the inner surface of the second inner hole to facilitate the movement of the medical device inside the inner hole.

In practical application, because the size of the endoscope is large and the guide tube is disposed within the expansion tube, the original working area of the expansion tube is reduced. When the endoscope is placed into the expansion tube, it almost occupies the entire minimally invasive channel and it is impossible to place other devices therein, therefore it cannot meet the requirement of simultaneous operation of multiple channels and multiple devices.

### SUMMARY

This application provides a multi-channel working cannula to solve the problem that the minimally invasive channel device in the prior art cannot adapt to the requirement of simultaneous operation of multiple channels and multiple devices.

The embodiments of the present disclosure model provide a multi-channel working cannula, which includes a main catheter and a plurality of accessory catheters, and the distance between the main catheter and the accessory catheters is adjustable; one end of the main catheter is provided with a guide plate, and the other end is an insertion end, and the guide plate extends in a direction perpendicular to the length of the main catheter;
the guide plate is provided with a first through hole, and the main catheter is inserted into the first through hole; the guide plate is provided with at least one elongated hole, and in case there is more than one elongated hole, the elongated holes are distributed at intervals along the plane in which the guide plate is located;
one end of the accessory catheter is provided with a mounting block, the length of the mounting block is less than that of the elongated hole, and the mounting block is inserted into the corresponding elongated hole;
the mounting block is provided with a second through hole, and the accessory catheter is inserted into the second through hole; the side of the mounting block is provided with a locking hole, the locking hole is in communication with the second through hole;
it also includes a first locking member;
the first locking member is inserted into the locking hole, and the accessory catheter is fixedly connected to the mounting block.

The guide plate is provided with positioning holes, and the positioning holes are distributed at intervals along both sides of the elongated hole;
the mounting block is provided with fixing holes;
it also includes a second locking member;
the second locking member is inserted into the fixing hole and the corresponding positioning hole to fix the mounting block on the guide plate.

Optionally, the mounting block is a T-shaped block;
the mounting block includes a first connecting portion and a second connecting portion, the second connecting portion protrudes from the first connecting portion;
the second through hole passes through the first connecting portion and the second connecting portion, and the locking hole is provided on the side of the first connecting portion;
the fixing hole is provided in a part where the second connecting portion protrudes from the first connecting portion.

Optionally, the width of the second connecting portion is greater than that of the elongated hole;
the width of the first connecting portion is equal to that of the elongated hole;
the height of the first connecting portion is equal to that of the elongated hole.

Optionally, the first locking member includes a first bolt and a first nut;
the first bolt is inserted into the locking hole, fixedly connecting the accessory catheter to the mounting block;
the second locking member includes a second bolt and a second nut;
the second bolt is inserted into the fixing hole and the corresponding positioning hole, fixing the mounting block onto the guide plate.

Optionally, the length of the first bolt is greater than that of the locking hole.

Optionally, the length of the second bolt is less than or equal to the sum of the thickness of the fixing hole and the positioning hole;
the mounting block is provided with a counterbore;
the length of the accessory catheter is less than that of the main catheter;
an insertion end of the main catheter is provided with an endoscope view hole on one side that is close to the accessory catheter, the endoscope view hole is an elongated hole and extends along the length direction of the main catheter.

Optionally, the second through hole on the mounting block is an inclined hole, and the second through hole is inclined from top to bottom in the direction of the main catheter.

Optionally, the main catheter and the guide plate are integrally arranged, and the insertion end of the main catheter is provided with an inclined opening on one side that is close to the accessory catheter.

Compared with the prior art, the present disclosure model has the following beneficial effects.
1. In the present disclosure model, a main catheter and a plurality of accessory catheters are included, and the distance between the main catheter and the accessory catheters is adjustable; one end of the main catheter is provided with a guide plate, and the other end is an insertion end, and the guide plate extends in a direction perpendicular to the length of the main catheter; the guide plate is provided with a first through hole, and the main catheter is inserted into the first through hole; the guide plate is provided with at least one elongated hole, and the elongated holes are distributed at intervals along the plane in which the guide plate is located; one end of the accessory catheter is provided with a mounting block, the length of the mounting block is less than that of the elongated hole, and the mounting block is inserted into the corresponding elongated hole; the mounting block is provided with a second through hole, and the accessory catheter is inserted into the second through hole; the side of the mounting block is provided with a locking hole, the locking hole is in communication with the second through hole; it also includes a first locking member; and the first locking member is inserted into the locking hole, fixedly connecting the accessory catheter to the mounting block. In the present disclosure model, by arranging an accessory catheter on the outside of the main catheter, that is, there is no interference to the function of the original main channel; at the same time, because one end of the accessory catheter is provided with a mounting block, it is needed only to move the mounting block along the length of the elongated hole to adjust the distance between the accessory catheter and the main catheter, preventing the insertion of larger devices in the main catheter from affecting the insertion of devices into the accessory catheter; because the accessory catheter and the main catheter are detachably connected, when it is needed to increase the accessory catheters, the accessory catheters can be inserted into the elongated holes of the guide plate through the mounting block. When the accessory catheters are not needed, the accessory catheters and the mounting blocks can be pulled out of the elongated holes of the guide plate, making the operation more flexible and convenient.
2. In the present disclosure model, the guide plate is provided with positioning holes, and the positioning holes are distributed at intervals along both sides of the elongated hole; the mounting block is provided with fixing hole; it also includes a second locking member; by arranging the fixing hole and the corresponding positioning holes, the second locking member is inserted into the fixing hole and the corresponding positioning hole to fix the mounting block on the guide plate, preventing the accessory catheter from shaking back and forth in the second through hole during the surgery, making the positioning of the devices inserted into the accessory catheter more accurate.
3. In the present disclosure model, the mounting block is a T-shaped block; the mounting block includes a first connecting portion and a second connecting portion, the second connecting portion protrudes from the first connecting portion; the second through hole passes through the first connecting portion and the second connecting portion, and the locking hole is provided on the side of the first connecting portion; and the fixing hole is provided in a part where the second connecting portion protrudes from the first connecting portion. The width of the second connecting portion is greater than that of the elongated hole; the width of the first connecting portion is equal to that of the elongated hole; the height of the first connecting portion is equal to that of the elongated hole, which can effectively restrict the slide of the mounting block along the length of the elongated hole, thereby aligning the fixing hole and the corresponding positioning hole, and facilitating the insertion of the second locking member into the fixing hole and the corresponding positioning hole, and facilitating the fixed connection of the mounting block with the guide plate.
4. In the present disclosure model, the length of the second bolt is less than or equal to the sum of the thickness of the fixing hole and the positioning hole; the mounting block is provided with a counter bore; the counter bore is arranged at the end of the first connecting portion away from the second connecting portion; the second nut is located in the counter bore to avoid scratching other appliances due to the second bolt being too long and protruding out of the outer surface of the mounting block.
5. In the present disclosure model, the length of the accessory catheter is less than that of the main catheter; the insertion end of the main catheter is provided with an endoscope view hole on one side that is close to the accessory catheter, the endoscope view hole is an elongated hole and extends along the length direction of the main catheter. During the actual operation, the condition of the devices inserted into the accessory catheter can be observed through the endoscope view hole on the main catheter, which facilitates the positioning of the devices in the accessory catheter and the surgical operation.
6. In the present disclosure model, the second through hole on the mounting block is an inclined hole, and the second through hole is inclined from top to bottom towards the direction of the main catheter, facilitating the insertion end of the accessory catheter to be close to the outer lateral wall of the main catheter during the surgery.
7. In the present disclosure model, the main catheter and the guide plate are integrally arranged, and the insertion end of the main catheter is provided with an inclined opening on one side that is close to the accessory catheter, which can guide the devices inserted into the main catheter, and facilitate the clamping and positioning of the main catheter and the affected area.

### DESCRIPTION OF DRAWINGS

In order to explain the background technology or the technical solutions of the present disclosure model more clearly, the accompanying drawings used in the prior art or in conjunction with the specific embodiments will be briefly introduced below. Obviously, the following accompanying drawings in conjunction with the specific embodiments are only used to facilitate the understanding of the embodiments of the present disclosure model. For those of ordinary skill in the art, other accompanying drawings can also be obtained based on these accompanying drawings without paying creative work.
Figure 1 is a schematic diagram of the overall assembly structure provided by an embodiment of the present disclosure model;
Figure 2 is a schematic side and cross-sectional structural view of the guide plate provided by an embodiment of the present disclosure model;
Figure 3 is a schematic top structural view of the guide plate provided by an embodiment of the present disclosure model;
Figure 4 is a schematic structural view of the mounting block provided by an embodiment of the present disclosure model;

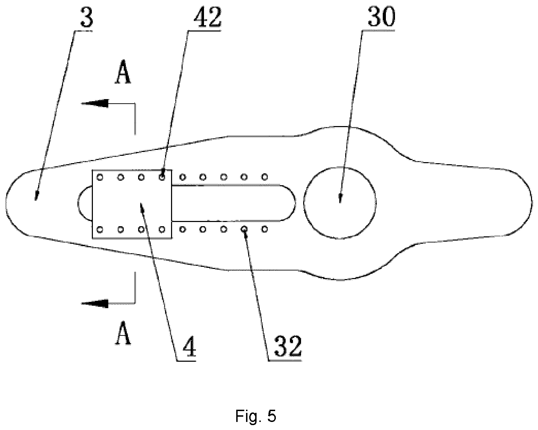

Figure 5 is a schematic top structural view of the assembly of the mounting block and the guide plate provided by an embodiment of the present disclosure model;

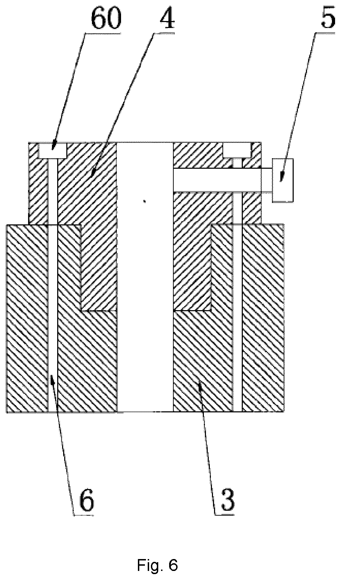

Figure 6 is a schematic cross-sectional structural view at A-A in Figure 5; and
Figure 7 is a schematic structural view of the assembly during a surgery provided by an embodiment of the present disclosure model;

Reference numerals: 1: main catheter, 2: accessory catheter, 3: guide plate, 30: first through hole, 31: elongated hole, 32: positioning hole, 4: mounting block, 40: second through hole, 41: locking hole, 42: fixing hole, 43: first connecting portion, 44: second connecting portion, 5: first locking member, 6: second locking member, 60: second nut, 7: endoscope view hole, 8: inclined opening.

### DETAILED DESCRIPTION

In order to enable those skilled in the art to better understand the technical solutions of the present disclosure model, the technical solutions in the embodiments of the present disclosure model will be clearly and completely described below in conjunction with the accompanying drawings in the embodiments of the present disclosure model. Obviously, the embodiments described are merely some of, rather than all of the embodiments of the present disclosure model. Based on the embodiments of the present disclosure model, all other embodiments obtained by those of ordinary skill in the art without any creative effort shall fall within the protection scope of the present disclosure model.

The multi-channel working cannula provided by the embodiments of the present invention includes a main catheter 1 and one accessory catheter 2. The distance between the main catheter 1 and the accessory catheter 2 is adjustable. Of course, in other embodiments, the number of the accessory catheters 2 can also be set to multiple according to actual demand.

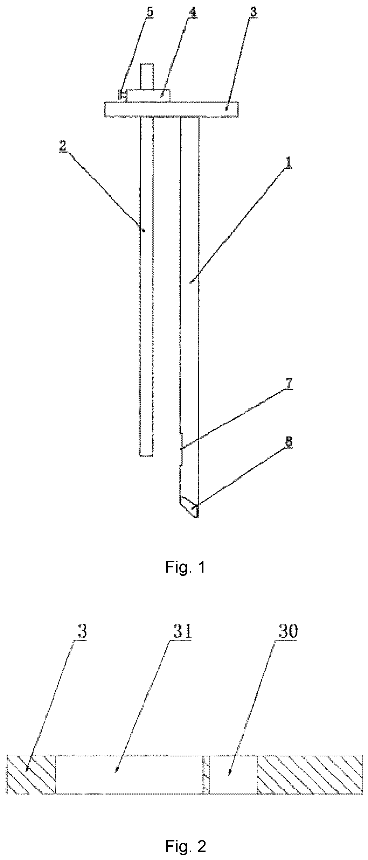

Figure 1 is a schematic structural view of the overall assembly provided by an embodiment of the present disclosure model. As shown in Figure 1, one end of the main catheter 1 is provided with a guide plate 3, the other end is an insertion end, and the guide plate 3 extends along the length direction perpendicular to the main catheter 1. In this embodiment, the main catheter 1 and the guide plate 3 are welded together. Of course, in other embodiments, the main catheter 1 and the guide plate 3 can also be integrally arranged, and the insertion end of the main catheter 1 is provided with an inclined opening 8 on one side that is close to the accessory catheter 2, which can guide the devices inserted into the main catheter 1, and facilitate the clamping and positioning of the main catheter 1 and the affected area.

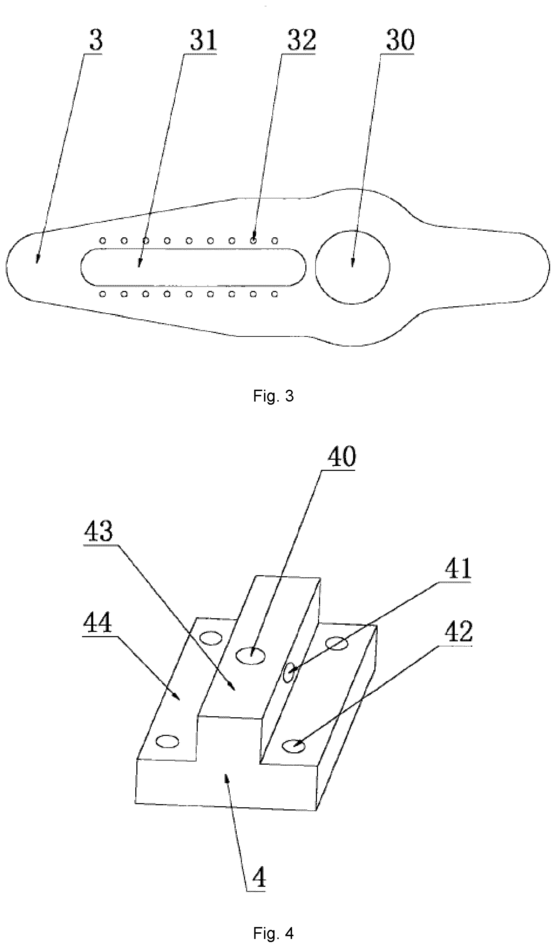

Figure 2 is a schematic side and cross-sectional structural view of the guide plate provided by an embodiment of the present disclosure model. Figure 3 is a schematic top structural view of the guide plate provided by an embodiment of the present disclosure model. As shown in Figures 2 and 3, the guide plate 3 is provided with a first through hole 30, and the main catheter 1 is inserted into the first through hole 30. The guide plate 3 is provided with at least one elongated holes 31, and the elongated holes 31 are distributed at intervals along the plane in which the guide plate 3 is located.

In this embodiment, the guide plate 3 is provided with positioning holes 32, and the positioning hole 32 are distributed at intervals along both sides of the elongated hole 31. The mounting block 4 is provided with fixing holes 42. It also includes a second locking member 6; the second locking member 6 is inserted into the fixing hole 42 and the corresponding positioning hole 32 to fix the mounting block 4 onto the guide plate 3.

By arranging the fixing holes 42 and the corresponding positioning holes 32, the second locking member 6 is inserted into the fixing hole 42 and the corresponding positioning hole 32 to fix the mounting block 4 onto the guide plate 3, preventing the accessory catheter 2 from shaking back and forth in the second through hole 40 during the surgery, making the positioning of the devices inserted into the accessory catheter 2 more accurate.

Figure 4 is a schematic structural view of the mounting block provided by an embodiment of the present disclosure model. As shown in Figure 4, the mounting block 4 is provided with a second through hole 40, and the accessory catheter 2 is inserted into the second through hole 40. The side of the mounting block 4 is provided with a locking hole 41, and the locking hole 41 is in communication with the second through hole 40.

It also includes a first locking member 5; and the first locking member 5 is inserted into the locking hole 41 to fixedly connecting the accessory catheter 2 to the mounting block 4.

In this embodiment, the mounting block 4 is a T-shaped block; the mounting block 4 includes a first connecting portion 43 and a second connecting portion 44, the first connecting portion 43 and the second connecting portion 44 are integrally provided, the second connecting portion 44 protrudes from the first connecting portion 43. The second through hole 40 passes through the first connecting portion 43 and the second connecting portion 44, and the locking hole 41 is provided on the side of the first connecting portion 43. The fixing hole 42 is provided in a part where the second connecting portion 44 protrudes from the first connecting portion 43.

The width of the second connecting portion 44 is greater than that of the elongated hole 31. The width of the first connecting portion 43 is equal to that of the elongated hole 31. The height of the first connecting portion 43 is equal to that of the elongated hole 31.

This can effectively restrict the slide of the mounting block 4 along the length of the elongated hole 31, thereby aligning the fixing hole 42 and the corresponding positioning hole 32, and facilitating the insertion of the second locking member 6 into the fixing hole 42 and the corresponding positioning hole 32, and facilitating the fixed connection of the mounting block 4 with the guide plate 3.

The first locking member 5 includes a first bolt and a first nut; the length of the first bolt is greater than that of the locking hole 41. The first bolt is inserted into the locking hole 41, and the accessory catheter 2 is fixedly connected to the mounting block 4.

The second locking member 6 includes a second bolt and a second nut 60. The first bolt is inserted into the fixing hole 42 and the corresponding positioning hole 32 to fix the mounting block 4 onto the guide plate 3.

In this embodiment, the length of the second bolt is less than or equal to the sum of the thickness of the fixing hole 42 and the positioning hole 32. The mounting block 4 is provided with counter bores; the counter bores are arranged at the end of the first connecting portion 43 away from the second connecting portion 44. The second nut 60 is located in the counter bore to prevent the second bolt from extending beyond the outer surface of the mounting block 4 and scratching other appliances due to its excessive length.

In this embodiment, the length of the accessory catheter 2 is less than that of the main catheter 1. The insertion end of the main catheter 1 is provided with an endoscope view hole 7 on one side that is close to the accessory catheter 2, and the endoscope view hole 7 is an elongated hole and extends along the length direction of the main catheter 1. During the actual operation, the condition of the devices inserted into the accessory catheter 2 can be observed through the endoscope view hole 7 on the main catheter 1, which facilitates the positioning of the devices in the accessory catheter 2 and the surgical operation.

In order to facilitate the observation of the insertion depth of the main catheter 1 and the accessory catheter 2, in this embodiment, scale lines and scale values are provided on the outer side walls of the main catheter 1 and the accessory catheter 2.

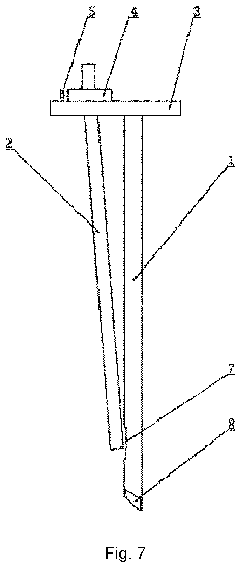

Figure 7 is a schematic structural view of the assembly during the surgery provided by an embodiment of the present disclosure model. As shown in Figure 7, in other embodiments, in order to facilitate the insertion end of the accessory catheter 2 to be close to the outer lateral wall of the main catheter 1 during the surgery, the second through hole 40 on the mounting block 4 is an inclined hole, and the second through hole 40 is inclined from top to bottom towards the direction of the main catheter 1.

During the actual operation, the endoscope can be inserted into the accessory catheter 2, and multiple devices (such as nerve root protection, vascular forcep, etc.) can be placed in the main catheter 1 at the same time, which is convenient for doctors to cooperatively use. Of course, in other embodiments, the endoscope can also be inserted into the accessory catheter 2. In the present disclosure model, by arranging an accessory catheter 2 on the outside of the main catheter 1, that is, there is no interference to the function of the original main channel. At the same time, because one end of the accessory catheter 2 is provided with a mounting block 4, it is only needed to move the mounting block 4 along the length of the elongated hole 31 to adjust the distance between the accessory catheter 2 and the main catheter 1, preventing the insertion of larger devices in the main catheter 1 from affecting the insertion of devices in the accessory catheter. Because the accessory catheter 2 and the main catheter 1 are detachably connected, when it is needed to increase the accessory catheters 2, the accessory catheters 2 can be inserted into the elongated holes 31 of the guide plate 3 through the mounting block 4. When the accessory catheters 2 are not needed, the accessory catheters 2 and the mounting blocks 4 can be pulled out of the elongated holes 31 of the guide plate 3, making the operation more flexible and convenient.

The multi-channel working cannula in the embodiments of the present disclosure model has been described in detail above. The principles and implementation of the present disclosure model will be illustrated in this section by use of specific embodiments. The description of the above embodiments is only used to facilitate the understanding of the core idea of the present disclosure model. Without departing from the principle of the present disclosure model, all other embodiments obtained by those of ordinary skill in the art without paying any inventive effort fall within the scope of the present disclosure model. However, the scope of the invention is solely defined by the appended claims.

## Claims

1. A multi-channel working cannula, comprising main catheter (1) and a plurality of accessory catheters (2), 2. wherein:
the distance between the main catheter (1) and the accessory catheters (2) is adjustable; one end of the main catheter (1) is provided with a guide plate (3), and the other end is an insertion end, and the guide plate (3) extends in a direction perpendicular to the length of the main catheter (1);
the guide plate (3) is provided with a first through hole (30), and the main catheter (1) is inserted into the first through hole (30); the guide plate (3) is provided with at least one elongated hole (31), and in case there is more than one elongated hole (31), the elongated holes (31) are at intervals distributed along the plane on which the guide plate (3) is located;
one end of the accessory catheter (2) is provided with a mounting block (4), the length of the mounting block (4) is less than that of the elongated hole (31), and the mounting block (4) is inserted into the corresponding elongated hole (31);
the mounting block (4) is provided with a second through hole (40), and the accessory catheter (2) is inserted into the second through hole (40);
**characterized in that**:
the side of the mounting block (4) is provided with a locking hole (41), the locking hole (41) is communicated with the second through hole (40);
it also includes a first locking member (5);
the first locking member (5) is inserted into the locking hole (41), and the accessory catheter (2) is fixedly connected to the mounting block (4), wherein the guide plate (3) is provided with positioning holes (32), and the positioning hole (32) are at intervals distributed along both sides of the elongated hole (31);
the mounting block (4) is provided with fixing holes (42);
it also includes a second locking member (6);
the second locking member (6) is inserted into the fixing hole (42) and the corresponding positioning hole (32) to fix the mounting block (4) on the guide plate (3).

2. The multi-channel working cannula according to claim 1, **characterized in that**:
the mounting block (4) is a T-shaped block;
the mounting block (4) comprises a first connecting portion (43) and a second connecting portion (44), the second connecting portion (44) protrudes from the first connecting portion (43);
the second through hole (40) passes through the first connecting portion (43) and the second connecting portion (44), and the locking hole (41)
is provided on the side of the first connecting portion (43);
the fixing hole (42) is arranged at a part where the second connecting portion (44) protruding from the first connecting portion (43).

3. The multi-channel working cannula according to claim 2, **characterized in that**:
the width of the second connecting portion (44) is greater than that of the elongated hole (31);
the width of the first connecting portion (43) is equal to that of the elongated hole (31);
the height of the first connecting portion (43) is equal to that of the elongated hole (31).

4. The multi-channel working cannula according to claim 3, **characterized in that**:
the first locking member (5) includes a first bolt and a first nut;
the first bolt is inserted into the locking hole (41), and the accessory catheter (2) is fixedly connected to the mounting block (4);
the second locking member (6) includes a second bolt and a second nut (60);
the second bolt is inserted into the fixing hole (42) and the corresponding positioning hole (32) to fix the mounting block (4) on the guide plate (3).

5. The multi-channel working cannula according to claim 4, **characterized in that**:
the length of the first bolt is greater than that of the locking hole (41).

6. The multi-channel working cannula according to claim 5, **characterized in that**:
the length of the second bolt is less than or equal to the sum of the thickness of the fixing hole (42) and the positioning hole (32); the mounting block (4) is provided with counter bored holes;
the counter bored holes are arranged at the end of the first connecting portion (43) away from the second connecting portion (44);
the second nut (60) is located in the counter bored hole.

7. The multi-channel working cannula according to claim 6, **characterized in that**:
the length of the accessory catheter (2) is less than that of the main catheter (1) ;
the insertion end of the main catheter (1) is provided with an endoscope view hole (7) on one side that is close to the accessory catheter (2), the endoscope view hole is an elongated hole and extends along the length direction of the main catheter (1).

8. The multi-channel working cannula according to claim 7, **characterized in that**:
the second through hole (40) on the mounting block (4) is an inclined hole, and the second through hole (40) is inclined from top to bottom in the direction of the main catheter (1).

9. The multi-channel working cannula according to claim 8, **characterized in that**:
the main catheter (1) and the guide plate (3) are integrally arranged, and the insertion end of the main catheter (1) is provided with an inclined opening (8) on one side that is close to the accessory catheter (2).

## Patentansprüche

1. Mehrkanalige Arbeitskanüle, die umfasst:
einen Hauptkatheter (1) und mehrere Zubehörkatheter (2), wobei:
der Abstand zwischen dem Hauptkatheter (1) und den Zubehörkathetern (2) anpassbar ist;
ein Ende des Hauptkatheters (1) mit einer Führungsplatte (3) versehen ist, und das andere Ende ein Einsatzende ist, und wobei sich die Führungsplatte (3) in eine Richtung erstreckt, die senkrecht zu der Länge des Hauptkatheters (1) ist;
die Führungsplatte (3) mit einem ersten Durchgangsloch (30) versehen ist, und der Hauptkatheter (1) in das erste Durchgangsloch (30) eingesetzt ist; wobei die Führungsplatte (3) mit mindestens einem langgestreckten Loch (31) versehen ist, und wobei für den Fall, dass mehr als ein langgestrecktes Loch (31) vorhanden ist, die langgestreckten Löcher (31) entlang der Ebene, auf der sich die Führungsplatte (3) befindet, in Intervallen verteilt angeordnet sind;
ein Ende des Zubehörkatheters (2) mit einem Befestigungsblock (4) versehen ist, wobei die Länge des Befestigungsblocks (4) kleiner als die des langgestreckten Lochs (31) ist, und wobei der Befestigungsblock (4) in das entsprechende langgestreckte Loch (31) eingesetzt ist;
der Befestigungsblock (4) mit einem zweiten Durchgangsloch (40) versehen ist, und der Zubehörkatheter (2) in das zweite Durchgangsloch (40) eingesetzt ist;
**dadurch gekennzeichnet, dass**
die Seite des Befestigungsblocks (4) mit einem Verriegelungsloch (41) versehen ist, wobei das Verriegelungsloch (41) mit dem zweiten Durchgangsloch (40) in Verbindung steht;
sie auch ein erstes Verriegelungselement (5) enthält;
das Verriegelungselement (5) in das Verriegelungsloch (41) eingesetzt ist, und der Zubehörkatheter (2) mit dem Befestigungsblock (4) fest verbunden ist, wobei
die Führungsplatte (3) mit Positionslöchern (32) versehen ist, und wobei die Positionslöcher (32) entlang beider Seiten des langgestreckten Loches (31) in Intervallen verteilt angeordnet sind;
der Befestigungsblock (4) mit Befestigungslöchern (42) versehen ist;
er auch ein zweites Verriegelungselement (6) enthält;
das zweite Verriegelungselement (6) in das Befestigungsloch (42) und das entsprechende Positionsloch (32) eingesetzt ist, um den Befestigungsblock (4) auf der Führungsplatte (3) zu befestigen.

2. Mehrkanalige Arbeitskanüle nach Anspruch 1, **dadurch gekennzeichnet, dass**:
der Befestigungsblock (4) ein T-förmiger Block ist;
der Befestigungsblock (4) einen ersten Verbindungsabschnitt (43) und einen zweiten Verbindungsabschnitt (44) umfasst, wobei der zweite Verbindungsabschnitt (44) von dem ersten Verbindungsabschnitt (43) hervorsteht,
das zweite Durchgangsloch (40) durch den ersten Verbindungsabschnitt (43) und den zweiten Verbindungsabschnitt (44) hindurch verläuft, und das das Verriegelungsloch (41)
auf der Seite des ersten Verbindungsabschnitts (43) bereitgestellt ist;
das Befestigungsloch (42) an einem Teil angeordnet ist, an dem der zweite Verbindungsabschnitt (44) von dem ersten Verbindungsabschnitt (43) hervorsteht.

3. Mehrkanalige Arbeitskanüle nach Anspruch 2, **dadurch gekennzeichnet, dass**:
die Breite des zweiten Verbindungsabschnitts (44) größer als die des langgestreckten Lochs (31) ist;
die Breite des ersten Verbindungsabschnitts (43) gleich der des langgestreckten Lochs (31) ist;
die Höhe des ersten Verbindungsabschnitts (43) gleich der des langgestreckten Lochs (31) ist.

4. Mehrkanalige Arbeitskanüle nach Anspruch 3, **dadurch gekennzeichnet, dass**:
das erste Verriegelungselement (5) einen ersten Bolzen und eine erste Mutter enthält;
die erste Mutter in das Verriegelungsloch (41) eingesetzt ist, und der Zubehörkatheter (2) mit dem Befestigungsblock (4) fest verbunden ist;
das zweite Verriegelungselement (6) einen zweiten Bolzen und eine zweite Mutter (60) enthält;
der zweite Bolzen in das Befestigungsloch (42) und das entsprechende Positionsloch (32) eingesetzt ist, um den Befestigungsblock (4) auf der Führungsplatte (3) zu befestigen.

5. Mehrkanalige Arbeitskanüle nach Anspruch 4, **dadurch gekennzeichnet, dass**:
die Länge des ersten Bolzens größer als die des Verriegelungslochs (41) ist.

6. Mehrkanalige Arbeitskanüle nach Anspruch 5, **dadurch gekennzeichnet, dass**:
die Länge des zweiten Bolzens kleiner oder gleich der Summe der Dicke des Befestigungslochs (42) und des Positionslochs (32) ist; der Befestigungsblock (4) mit Schulterbohrungen versehen ist;
die Schulterbohrungen an dem Ende des ersten Verbindungsabschnitts (43) abgewandt von dem zweiten Verbindungsabschnitt (44) angeordnet sind;
die zweite Mutter (60) in der Schulterbohrung angeordnet ist.

7. Mehrkanalige Arbeitskanüle nach Anspruch 6, **dadurch gekennzeichnet, dass**:
die Länge des Zubehörkatheters (2) kleiner als die des Hauptkatheters (1) ist;
das Einsatzende des Hauptkatheters (1) mit einem Endoskop-Sichtloch (7) auf einer Seite versehen ist, die nahe bei dem Zubehörkatheter (2) gelegen ist, wobei das Endoskop-Sichtloch ein langgestrecktes Loch ist und sich entlang der Längsrichtung des Hauptkatheters (1) erstreckt.

8. Mehrkanalige Arbeitskanüle nach Anspruch 7, **dadurch gekennzeichnet, dass**:
das zweite Durchgangsloch (40) auf dem Befestigungsblock (4) ein geneigtes Loch ist, und dass das zweite Durchgangsloch (40) von oben nach unten in der Richtung des Hauptkatheters (1) geneigt ist.

9. Mehrkanalige Arbeitskanüle nach Anspruch 8, **dadurch gekennzeichnet, dass**:
der Hauptkatheter (1) und die Führungsplatte (3) einstückig angeordnet sind, und dass das Einsatzende des Hauptkatheters (1) mit einer geneigten Öffnung (8) auf einer Seite versehen ist, die nahe bei dem Zubehörkatheter (2) gelegen ist.

## Revendications

1. Canule de travail multi-voies, comprenant
un cathéter principal (1) et une pluralité de cathéters accessoires (2),
dans laquelle :
la distance entre le cathéter principal (1) et les cathéters accessoires (2) est réglable ; une extrémité du cathéter principal (1) est dotée d'une plaque de guidage (3) et l'autre extrémité est une extrémité d'insertion, et la plaque de guidage (3) s'étend dans une direction perpendiculaire à la longueur du cathéter principal (1) ;
la plaque de guidage (3) est dotée d'un premier trou traversant (30) et le cathéter principal (1) est inséré dans le premier trou traversant (30) ; la plaque de guidage (3) est dotée d'au moins un trou allongé (31), et au cas où il y a plus d'un trou allongé (31), les trous allongés (31) sont répartis à intervalle le long du plan sur lequel la plaque de guidage (3) est située ;
une extrémité du cathéter accessoire (2) est dotée d'un bloc de montage (4) la longueur du bloc de montage (4) est inférieure à celle du trou allongé (31), et le bloc de montage (4) est inséré dans le trou allongé correspondant (31) ;
le bloc de montage (4) est doté d'un second trou traversant (40), et le cathéter accessoire (2) est inséré dans le second trou traversant (40) ;
**caractérisée en ce que**
le côté du bloc de montage (4) est doté d'un trou de blocage (41), le trou de blocage (41) communique avec le second trou traversant (40) ;
il inclut également un premier membre de blocage (5) ;
le premier membre de blocage (5) est inséré dans le trou de blocage (41) et le cathéter accessoire (2) est relié fixement au bloc de montage (4), dans laquelle
la plaque de guidage (3) est dotée de trous de positionnement (32), et les trous de positionnement (32) sont répartis à intervalle le long des deux côtés du trou allongé (31) ;
le bloc de montage (4) est doté de trous de fixation (42) ;
il inclut également un second membre de blocage (6) ;
le second membre de blocage (6) est inséré dans le trou de fixation (42) et le trou de positionnement (32) correspondant pour fixer le bloc de montage (4) sur la plaque de guidage (3).

2. Canule de travail multi-voies selon la revendication 1, **caractérisée en ce que** :
le bloc de montage (4) est un bloc en forme de T ;
le bloc de montage (4) comprend une première partie de connexion (43) et une seconde partie de connexion (44), la seconde partie de connexion (44) fait saillie de la première partie de connexion (43) ;
le second trou traversant (40) passe à travers la première partie de connexion (43) et la seconde partie de connexion (44), et le trou de blocage (41) est prévu sur le côté de la première partie de connexion (43) ;
le trou de fixation (42) est agencé sur une partie où la seconde partie de connexion (44) fait saillie de la première partie de connexion (43).

3. Canule de travail multi-voies selon la revendication 2, **caractérisée en ce que** :
la largeur de la seconde partie de connexion (44) est supérieure à celle du trou allongé (31) ;
la largeur de la première partie de connexion (43) est égale à celle du trou allongé (31) ;
la hauteur de la première partie de connexion (43) est égale à celle du trou allongé (31).

4. Canule de travail multi-voies selon la revendication 3, **caractérisée en ce que** :
le premier membre de blocage (5) inclut un premier boulon et un premier écrou ;
le premier boulon est inséré dans le trou de blocage (41), et le cathéter accessoire (2) est relié fixement au bloc de montage (4) ;
le second membre de blocage (6) inclut un second boulon et un second écrou (60) ;
le second boulon est inséré dans le trou de fixation (42) et le trou de positionnement (32) correspondant pour fixer le bloc de montage (4) sur la plaque de guidage (3).

5. Canule de travail multi-voies selon la revendication 4, **caractérisée en ce que** :
la longueur du premier boulon est supérieure à celle du trou de blocage (41).

6. Canule de travail multi-voies selon la revendication 5, **caractérisée en ce que** :
la longueur du second boulon est inférieure ou égale à la somme de l'épaisseur du trou de fixation (42) et du trou de positionnement (32) ; le bloc de montage (4) est doté de trous à contre-perçage ;
les trous à contre-perçage sont agencés à l'extrémité de la première partie de connexion (43) à distance de la seconde partie de connexion (44) ;
le second écrou (60) est situé dans le trou à contre-perçage.

7. Canule de travail multi-voies selon la revendication 6, **caractérisée en ce que** :
la longueur du cathéter accessoire (2) est inférieure à celle du cathéter principal (1) ;
l'extrémité d'insertion du cathéter principal (1) est dotée d'un orifice de vue d'endoscope (7) sur un côté qui est proche du cathéter accessoire (2), l'orifice de vue d'endoscope est un orifice allongé et s'étend le long de la direction en longueur du cathéter principal (1).

8. Canule de travail multi-voies selon la revendication 7, **caractérisée en ce que** :
le second trou traversant (40) sur le bloc de montage (4) est un trou incliné, et le second trou traversant (40) est incliné du haut vers le bas dans la direction du cathéter principal (1).

9. Canule de travail multi-voies selon la revendication 8, **caractérisée en ce que** :
le cathéter principal (1) et la plaque de guidage (3) sont agencés intégralement, et l'extrémité d'insertion du cathéter principal (1) est dotée d'une ouverture inclinée (8) sur un côté qui est proche du cathéter accessoire (2).
